## Europäisches Patentamt

(19) ))) **European Patent Office**

**Office européen des brevets**

(11) Numéro de publication: **0 188 953**

**B1**

(12) **FASCICULE DE BREVET EUROPÉEN**

(45) Date de publication du fascicule du brevet: **12.10.88**

(21) Numéro de dépôt: **85402559.0**

(22) Date de dépôt: **19.12.85**

(51) Int. Cl.⁴: **A 23 K 1/00,** A 23 K 1/18, A 61 K 9/52, A 61 K 9/50

(54) Compositions pour l'enrobage des additifs alimentaires destinés aux ruminants et additifs alimentaires ainsi enrobés.

(30) Priorité: 20.12.84 FR 8419520
20.12.84 FR 8419521

(43) Date de publication de la demande:
30.07.86 Bulletin 86/31

(45) Mention de la délivrance du brevet:
12.10.88 Bulletin 88/41

(84) Etats contractants désignés:
AT BE CH DE FR GB IT LI LU NL SE

(56) Documents cité:
EP-A-0 068 777
FR-A-2 081 320
FR-A-2 401 620
FR-A-2 401 621
FR-A-2 498 084
FR-A-2 506 613
FR-A-2 514 261
FR-A-2 524 269
FR-A-2 541 305

CHEMICAL ABSTRACTS, vol. 90, no. 21, 21 mai 1979, page 220, abrégé no.164048f, Columbus, Ohio, US

(73) Titulaire: RHONE- POULENC SANTE, Les Miroirs 18 Avenue d'Alsace, F-92400 Courbevoie Cédex (FR)

(72) Inventeur: Autant, Pierre, 14 rue de Pourcheroux, F-03600 Commentry (FR)
Inventeur: Cartillier, André, Champfromenteau, F-03600 Commentry (FR)
Inventeur: Pigeon, Raymond, 52 Chemin Moulin du Got, F-69340 Francheville (FR)

(74) Mandataire: Pilard, Jacques, RHONE- POULENC RECHERCHES Service Brevets Pharma 25, Quai Paul Doumer, F-92408 Courbevoie Cedex (FR)

EP 0 188 953 B1

# 0 188 953

**Description**

La présente invention concerne de nouvelles compositions pour l'enrobage des additifs alimentaires destinés aux ruminants qui sont stables dans un milieu dont le pH est égal ou supérieur à 5,5 et qui permet la libération de l'additif alimentaire dans un milieu dont le pH est inférieur ou égal à 3,5.

En particulier, lorsqu'on administre à des ruminants certaines substances biologiquement actives (médicaments, aliments enrichis), il se produit lors du passage dans le rumen une destruction enzymatique de ces substances favorisée par le temps de séjour (quelques heures à plusieurs jours) et par le pH (compris entre 5 et 6).

Il importe donc de pouvoir protéger ces substances biologiquement actives par des enrobages qui soient stables à un pH supérieur ou égal à 5, c'est-à-dire qui soient stables dans la panse des ruminants qui résistent à la dégradation par les microorganismes et qui permettent la libération des substances biologiquement actives dans une partie de l'appareil digestif, plus particulièrement dans la caillette, dont le pH est inférieur ou égal à 3,5. Alors que la durée de protection dans la panse doit être relativement longue (plusieurs heures à quelques jours), la libération de la substance active dans la caillette doit s'effectuer en un temps relativement court (de quelques minutes à 1 ou 2 heures).

Pour obtenir de tels résultats, il est avantageux de pouvoir disposer d'enrobages pour les substances actives dont la structure et la composition sont telles qu'ils sont insolubles dans le rumen à un pH compris entre 5 et 6, mais solubles, dispersés ou fortement gonflés dans la caillette à un pli inférieur à 3,5 pour libérer la substance active.

Il a été proposé, pour la réalisation de tels enrobages, d'utiliser, entre autres, des copolymères de l'anhydride maléïque avec un autre monomère, modifiés par action d'une diamine primaire - tertiaire sur les groupements anhydrides, formant ainsi des groupes imides aminés qui apportent la solubilité souhaitée (brevet français FR-1 536 774). Des dérivés cellulosiques aminés sont égalelement connus ; ils sont obtenus à partir d'un dérivé non saturé de la cellulose (éther, ester) sur lequel on fait réagir un composé azoté contenant un atome d'hydrogène mobile, tel que la pipéridine, la morpholine ou une amine secondaire (brevet français FR-6 930 562/2 081 320).

Par ailleurs, dans les brevets anglais GB-1 137 214, australien AU-454 117 et belge BE-865 654 et la demande sud-africaine SA-7 004 813 ainsi que dans les brevets français FR-7 433 108/2 246 572 et américain US 3 341 505 sont décrits des copolymères de:

a) un monomère éthylénique neutre comme l'acrylate ou le méthacrylate de méthyle, le styrène, l'acrylonitrile, l'acétate de vinyle, et

b) un monomère déthylénique portant un groupement azoté basique comme l'acrylate ou le méthacrylate de diéthylaminoéthyle, l'acrylate ou le méthacrylate de t. butylaminoéthyle, le méthacrylate de morpholinoéthyle ou les vinylpyridines.

Pour enrober des aliments destinés à la nourriture des ruminants, il a été proposé d'utiliser des copolymères styrènevinylpyridine contenant des substances hydrophobes choisies parmi les acides gras contenant 10 à 32 atomes de carbone, des acides polycarboxyliques comprenant 10 à 2, atomes de carbone par groupe carboxyle qui améliorent la protection en diminuant la susceptibilité globale de la pellicule d'enrobage aux milieux aqueux de caractère faiblement acide (brevet français FR-7 823 966/2 401 620). Dans de telles compositions d'enrobage, la substance hydrophobe permet de diminuer la mouillabilité du polymère mais elle reste sans influence sur la libération du principe actif en milieu acide.

Dans le brevet français FR-8 118 954/2 5142 61 a été décrit oun enrobage constitué d'un copolymère sensible aux variations du pH, choisi parmi les copolymères du styrène avec les vinylpyridines, et d'un polymère non hydrosoluble insensible aux variations du pH, choisi parmi l'acétobutyrate de cellulose, l'éthylcellulose et le propionate de cellulose, ce dernier favorisant la libération de la substance active à un pH compris entre 1 et 2,5 et permettant de diminuer l'extractibilité de la substance active en milieu aqueux. Dans ces compositions, le polymère non hydrosoluble représente 10 à 75 % en poids du melange des polymères, et dans le cas de l'acetobutyrate de cellulose de 20 à 40 %.

Dans le brevet français FR-7 823 968 (2 401 621) a été décrite l'utilisation d'un polymère hydrophobe dans lequel est dispersée une substance soluble en milieu acide (phosphates alcalins, polymères basiques réticulés). Cependant il n'est pas établi que le liant, pour être efficace, doit posséder une hydrophilie contrôlée qui, éventuellement, selon la nature de la charge sensible, doit être fonction du pH. Dans ces compositions, la substance dispersée represente 40 à 60 % du volume de l'enrobage.

Il a maintenant été trouvé, et c'est ce qui fait l'objet de la présente invention, que l'on peut enrober efficacement des substances actives en vue de les administrer à des ruminants au moyen d'une composition constituée essentiellement d'un système liant, à hydrophilie contrôlée, éventuellement légèrement sensible aux variations du pH associé à une ou plusieurs substances très sensibles aux variations du pH, compatibles ou incompatibles avec le système liant.

Dans le cadre de ta présente invention, il est entendu que la différence de sensibilité au pH doit se situer dans une zone comprise entre pH = 1 et pH = 7. La sensibilité au pH peut être caractérisée par une modification des propriétés physiques et/ou chimiques des substances entre des valeurs du pH supérieures ou égales à 5 et inférieures ou égales à 3,5. La modification considérée est variable selon les substances. Dans le

2

cas d'un polymère, il peut s'agir du gonflement qui peut aller jusqu'à une solubilité totale ou de la vitesse de diffusion de l'eau ou de solutions aqueuses. Dans le cas de substances solides minérales ou organiques, il peut s'agir de la variation de la solubilité ou de la vitesse de solubilisation. Dans le cas de substances liquides, il peut s'agir de variation de la miscibilité. Une légère sensibilité aux variations du pH dit système liant signifie que les propriétés du liant sont affectées par le milieu lorsque le pH devient inférieur ou égal à 3,5 sans pour autant que les modifications induites soient directement responsables de la liberation massive de la substance active enrobée.

Pour la réalisation des compositions d'enrobage selon l'invention le système liant à hydrophilie contrôlée et éventuellement légèrement sensible aux variations du pH, dans le domaine considéré, résulte de l'association:

- d'un élément filmogène assurant la cohésion de l'ensemble,
- d'un élément de contrôle de la balance hydrophile/hydrophobe,
- éventuellement d'un élément légèrement pH sensible,

chaque partie du liant pouvant remplir plusieurs fonctions simultanément par exemple filmogène et légèrement pH sensible ou hydrophobe et légèrement pH sensible.

L'élément filmogène est choisi parmi les polymères naturels ou synthétiques susceptibles de former un film cohérent non hydrosoluble tels que des esters ou des esters cellulosiques comme l'éthyl cellulose, l'acétate, le propionate ou l'acétobutyrate de cellulose, des dérivés vinyliques comme l'acétate de polyvinyle, ainsi que des protéines non hydrosolubles comme la zéine. La zéine qui est isolée du gluten de maïs présente en particulier l'intérêt d'être à la fois l'élément filmogène nécessaire à la tenue mécanique de l'enrobage et un élément légèrement pH sensible utile au déclenchement du processus de libération contrôlée du principe actif enrobé en milieu acide, compte tenu de son point isoélectrique voisin de 6,2.

L'élément de contrôle de l'hydrophilie est, selon la nature du système liant utilisé, soit un produit hydrophobe tel qu'un polymère hydrophobe polyacétate de vinyle, dérivé cellulosique non hydrophobe, soit un dérivé hydrophile ou hydrosoluble tel qu'un polyol tel que le glycérol, l'éthylène glycol, le propylène glycol ou le dipropylène glycol.

L'élément légèrement pH sensible est choisi parmi les substances non hydrosolubles à fonctions basiques, qui apportent au système liant une affinité pour les milieux acides mais qui n'entraînent ni la dissolution ni la perte de cohésion du liant, telles des protéines hydrophobes à point isoélectrique suffisamment élevé comme la zéine.

D'un intérêt tout particulier est un système liant constitué de zéine qui peut être utilisée seule ou associé à des substances non hydrosolubles et filmogène choisies parmi les polymères tels que le polyacétate de vinyle et les dérivés cellulosiques non hydrosolubles.

Il est particulièrement avantageux d'utiliser des systèmes liants constitués de zéine associée à des dérivés non hydrosolubles de la cellulose tel que l'éthylcellulose ou l'acétobutyrate de cellulose.

Dans un tel système la proportion pondérale de polymère filmogène est généralement supérieure à 50 %.

Les substances très sensibles aux variations du pH peuvent être d'origines diverses. Elles sont généralement choisies parmi les polymères et copolymères basiques et les polyglycosamines. Parmi les polymères et copolymères basiques peuvent être cités les polymères ou copolymères contenant au moins un groupement amino basique et dont la teneur en azote est comprise entre 2 et 14 % tels que les dérivés aminés de la cellulose, les polymères et copolymères des dérivés aminés des acides acrylique, methacrylique et crotonique et les polymères ou copolymères du styrène ou de l'acrylonitrile avec les isomères ou les dérivés de la vinylpyridine tels que la vinyl-2 pyridine, la vinyl-4 pyridine ou le méthyl-2 vinyl-5 pyridine et les polymères et copolymères des acrylates et méthacrylates N-alcoylés. Parmi les polyglucosamines peut être cite tout particulièrement le chitosan obtenu par desacétylation de la chitine que l'on trouve en abondance dans les carapaces de crustacés.

Les substances très sensibles aux variations du pH sont utilisées soit en solution, soit sous forme de poudre dont la granulométrie varie selon l'épaisseur de la pellicule enrobante que l'on désire obtenir.

Dans la composition d'enrobage selon la présente invention, il est nécessaire de réaliser un équilibre entre les divers constituants je telle manière que:

- en milieu neutre ou légèrement acide, le principe actif ne soit pas libéré du fait de la limitation de l'hydrophilie,
- en milieu acide, les charges très sensibles aux variations du pH soient accessibles après une lente diffusion du milieu rendue possible du fait de l'hydroplilie contrôlée du liant et éventuellement favorisée par la présence de la substance légèrement sensible aux variations du pH. La dissolution ou le gonflement de la substance très sensible aux variations du pH assure ensuite la dégradation de l'enveloppe par gonflement, délitement ou éclatement et la libération du principe actif.

Dans la composition d'enrobage selon la présente invention, la quantité de liant représente généralement de 40 à 95 % en poids de la composition et la quantité de la substance très sensible aux variations du pH représente 5 à 50 % en volume de la composition.

D'un intérêt tout particulier sont les compositions d'enrobage contenant de la zéine, de l'éthylcellulose et un polymère ou copolymère basique dans des proportions telles que la texture de la couche enrobante obtenue présente une porphologie à domaine de microphase, la teneur en polymère ou copolymère basique étant inférieure à 10 % en poids.

Sont également particulièrement intéressantes les compositions d'enrobage contenant de la zéine, de l'éthylcellulose et du chitosan.

Selon la présente invention, les compositions d'enrobage, en dehors du liant et de la substance très sensible aux variations du pH, peuvent contenir des adjuvants dont le rôle est de faciliter la mise en oeuvre des techniques de préparation de ces compositions ou d'améliorer leur caractéristiques physico-chimiques. Il peut être avantageux d'ajouter des agents plastifiants (triacétine), des agents lubrifiants (stéarate de magnésium), des agents antistatiques (triglycérides à chaînes polyosyéthylénées), des agents anti-mottants (silice, carbonate de calcium), des agents fongicides, des agents émulsifiants (esters de sorbitan oxyéthylénés, sucroglycérides), des agents de compatibilisation (gommes naturelles ou semi-naturelles comme des polysaccharides tels que les alginates, la gomme adragante, les pectines, les carraghénates, la gomme xanthane), des éthers cellulosiques (carboxyméthyl-, méthyl-, hydroxypropyl cellulose) ou des charges telles que des sels minéraux, du sucre, de l'amidon ou des protéines. Ces divers adjuvants ne représentent généralement que quelques pourcents en poids de la composition d'enrobage.

Les compositions selon l'invention peuvent être obtenues en dispersant ou en dissolvant la substance très sensible aux variations du pH éventuellement associée à un adjuvant dans une solution ou une dispersion du liant dans un solvant organique ou dans un mélange de solvants organiques convenables choisis en fonction de la nature spécifique du liant. Généralement, la composition d'enrobage est obtenue après évaporation du ou des solvants.

Dans certains cas, pour la mise en oeuvre de substances organiques ou minerales sensibles aux variations du pH sous forme pulvérulente, il peut être avantageux d'effectuer un pré-enrobage avec des adjuvants décrits ci-dessus dans le but d'adapter l'interface charge-liant pour améliorer, par exemple, l'étanchéité du système en milieu neutre ou pour la diminuer en milieu acide.

Pour améliorer l'étanchéité du système en milieu neutre, en particulier pour les substances actives très hydrosolubles, il est avantageux d'utiliser des charges lamellaires de faible granulométrie, telles que le mica, le talc ou l'aluminium, éventuellement prétraités par un agent hydrophobe tel que l'acide stéarique.

Les compositions d'enrobage selon la présente invention sont particulièrement utiles pour protéger des substances thérapeutiques ou nutritives diverses telles que des médicaments, des vitamines ou des aminoacides destinés à être administrés par voie orale à des ruminants. Ces substances enrobées sont généralement mélangées à la nourriture des animaux. Les substances thérapeutiques ou nutritives peuvent se présenter sous forme solide ou liquide.

Les substances enrobées sont de préférence des granulés sous forme de microcapsules constitués d'un noyau central entouré d'une pellicule continue de la composition d'enrobage. Cependant les substances actives peuvent aussi être dispersées dans la composition d'enrobage. En général, la composition d'enrobage représente 5 à 60 % en poids de granulé ou de la dispersion.

La présente invention concerne également les substances biologiquement actives enrobées ou dispersées dans une composition d'enrobage décrite précédemment.

Les granulés peuvent être obtenus par application des techniques connues. Selon la nature de la composition d'enrobage, et plus particulièrement de celle du liant non hydrosoluble, on utilise soit des techniques d'extrusion ou de pulvérisation de solutions ou d'émulsions en lit fluidisé, soit des techniques d'encapsulation en milieu fondu ou semi-fondu, soit des techniques d'enrobage en milieu liquide telle que la coacervation.

La présente invention concerne également l'encapsulation d'un noyau liquide constitué, par exemple, d'une solution concentrée de la substance active dans l'eau ou dans un solvant organique approprié.

Les granulés obtenus selon la présente invention sont stables au stockage et lors des manipulations, ne se détériorent pas lors de la confection des aliments pour animaux et ne sont pas détruits lors de leurs absorption par les animaux et en particulier par écrasement ou par broyage lors de la mastication.

La taille des granulés sera fonction de l'utilisation qui doit en être faite et plus particulièrement elle sera déterminée en fonction de l'animal auquel ils sont destinés.

Il est possible d'enrober des substances actives pour obtenir des granulés dont la taille est comprise entre 0,1 et 5 mm.

D'un intérêt tout particulier sont les granulés qui contiennent comme substances actives la méthionine, la lysine ou des vitamines (vitamine A) dont le rôle est très important dans l'alimentation des animaux et plus spécialement des ruminants.

Pour mettre en évidence la sensibilité des compositions d'enrobage selon l'invention aux variations du pH, des tests sont utilisés qui permettent de mesurer le relargage de la matière active en fonction du temps à différentes valeurs du pH et, notamment, à pH = 6 et à pH = 2, ou à pH = 5 et à pH = 1,5.

Un test consiste à réaliser dans un film de la composition d'enrobage un disque sur la face supérieure duquel est placée une quantité connue de matière active, l'autre face étant en contact avec la surface d'une solution aqueuse tamponnée agitée magnétiquement. La quantité de substance active relarguée dans la solution aqueuse est déterminée à partir de prélèvements, le même échantillon étant testé d'abord à pH = 6 puis à pH = 2.

Un autre test consiste à placer entre 2 disques identiques de la composition d'enrobage une quantité connue de substance active. Cet ensemble, dont l'étanchéité est assurée par serrage des bords, est immergé dans une quantité déterminée d'une solution aqueuse tamponnée agitée magnétiquement. La quantité de substance active relarguée est contrôlée à partir du prélèvement à pH = 6 puis consécutivement à pH = 2.

Le relargage de la substance active sous forme de granulés ou de microcapsules est examiné en agitant, dans des conditions déterminées, une quantité connue (le granulés ou de microcapsules dans le milieu tamponné maintenu à pH constant à une température de 40°C. On compare les vitesses de relargage d'un échantillon soumis à différentes valeurs du pH, notamment à pH = 6 et à pH = 2.

Les exemples suivants, donnés à titre non limitatif, illustrent les compositions d'enrobage selon la présente invention ainsi que leur utilisation pour la préparation de matières actives enrobées.

## Exemple 1 (comparatif)

Suivant la technique du lit fluidisé on enrobe 200 g de méthionine préalablement granulée sous forme de particules sphériques titrant 98 % dont le diamètre est compris entre 0,5 et 0,6 mm par une solution constituée de:

| | |
|---|---|
| · Acétobutyrate de cellulose | 45 g |
| · Copolymère vinyl-2 pyridine-styrène (70 - 30) | 15 g |
| · Tétrahydrofuranne | 600 cm$^3$ |

On obtient par pelliculage 260 g de produit enrobé titrant 70 % en méthionine.

Des essais de relargage sont effectués en milieu aqueux à pH = 2 et à pH = 6, à 40°C, sous agitation, à des intervalles de temps différents.

Les résultats sont donnés dans le tableau 1.

## Exemple 2

On opère comme dans l'exemple 1 mais en pulvérisant la solution suivante

| | |
|---|---|
| · Acétobutyrate de cellulose | 22,5 g |
| · Dipropylèneglycol (propanediol-1,2) | 22,5 g |
| · Copolymère vinyl-2 pyridine-styrène (70 - 30) | 15 g |
| · Tétrahydrofuranne | 600 cm$^3$ |

Des essais de relargage sont effectués en milieu aqueux à pH = 2 et à pH = 6, à 40°C, sous agitation, à des intervalles de temps différents.

Les résultats sont rassemblés dans le tableau 1.

### Tableau 1

| Exemples | Relargage à pH = 6 % après | | | | vitesse de relargage g/s | Relargage à pH = 2 % après | | | | vitesse de relargage g/s |
|---|---|---|---|---|---|---|---|---|---|---|
| | 1 h | 2 h | 3 h | 5 h | | 30 mn | 1 h | 2 h | 5 h | |
| 1 | 3 | 5 | 7 | 9 | 2,5.10$^{-5}$ | | 4 | 5 | 10 | 2,5.10$^{-5}$ |
| 2 | 5 | 9 | 13 | 21 | 6,4.10$^{-5}$ | 100 | | | | >3.10$^{-3}$ |

Ces résultats montrent l'action favorable de l'adjonction d'un adjuvant hydrophile hydrosoluble.

## Exemple 3

A un mélange constitué de 10 cm$^3$ d'éthanol et de 10 cm$^3$ de dichlorométhane on ajoute 5 g de zéine, 0,5 g d'éthylcellulose et 0,5 g de chitosan dont la granulométrie est de 40 à 63 microns et dont le taux de groupement NH$_2$ est de 7 %.

On coule sur une plaque de verre horizontale un film de 200 microns d'épaisseur. Après 1 heure de séchage à 40°C sous pression réduite (20 mm de mercure ; 2,7 kPa) on obtient un film opaque de 50 microns d'épaisseur On découpe deux disques de 30 mm de diamètre entre lesquels on place 100 mg de méthionine (qualité feed-grade). L'ensemble, dont l'étanchéité est assurée par serrage (ur les bords, est immergé dans 150 cm$^3$ d'une

solution aqueuse tamponnée agitée magnétiquement. La quantité de méthionine relarguée est contrôlée sur des prélèvements.

Les quantités de méthionine relarguée à pH = 6 puis consécutivement à pH = 2 sont données dans le tableau 2.

**Tableau 2**

| Exemple | Relargage à pH = 6 après 24 heures mg méthionine | Relargage à pH = 2 | |
|---|---|---|---|
| | | après 2 h. : mg méthionine | après 5 h. mg méthionine |
| 3 | 13 | 27 | 73 |

**Exemple 4**

Selon la technique du lit fluidisé, on enrobe 350 g de méthioninie, préalablement granulée sous forme de particules sphériques titrant 98 % dont le diamètre est compris entre 0,6 et 1 mm, par une solution/dispersion dont la composition est la suivante:

| | |
|---|---|
| Zéine | 80 g |
| Acétate de polyvinyle | 30 g |
| Ethylcellulose | 10 g |
| Chisotan (granulométrie : 40/80 microns ; taux de groupements NH$_2$ : 7 %) | 10 g |
| Triacétine | 10 g |
| Dichloro-1,2 éthane | 500 cm$^3$ |
| Ethanol | 500 cm$^3$ |
| Antistatique (Labrasol, marque déposée Gattefosse) | 3 cm$^3$. |

Par simple pelliculage, on obtient en 5 heures des granulés enrobés titrant 86 % en méthionine.

Le relargage de la méthionine est déterminé en dispersant 200 mg de granulés dans 200 cm$^3$ d'une solution tamponnée agitée magnétiquement à 300 tours/minute. La quantité de méthionine relarguée est contrôlée sur des prélèvements, l'essai étant fait sur des échantillons distincts à pH = 6 et à pH = 2.

Les résultats sont rassemblés dans le tableau 3.

**Exemple 5**

On effectue l'enrobage de la méthionine avec une composition enrobante identique à celle utilisée dans l'exemple 4 mais ne contenant que 5 g de chitosan dont la granulométrie est inférieure a 80 microns et dont le taux en groupements NH$_2$ est de 7 %.

On utilise une cuve équipée d'un système Wurster.

Après 3 heures, on obtient des granulés titrant 72,5 % en méthionine.

Les résultats sont rassemblés dans le tableau 3.

**Exemple 6** (comparatif)

On opère comme dans l'exemple 5 mais en utilisant une formulation enrobante ne contenant pas de substance sensible aux variations du pH et ayant la composition suivante:

| | |
|---|---|
| Zéine | 120 g |
| Triacétine | 10 g |
| Dichloro-1,2 éthane | 500 cm$^3$ |
| Ethanol | 500 cm$^3$ |
| Antistatique (Labrasol) | 3 cm$^3$ |

On obtient des granulés titrant 72,5 % en méthionine.

Les résultats sont rassemblés dans le tableau 3.

## Tableau 3

| Exemples | Titre en méthionine % | Relargage à pH = 6 après 24 heures % | Relargage à pH = 2 | |
|---|---|---|---|---|
| | | | après 2 h. % | après 5 h. % |
| 4 | 86 | 85 | 76 | 100 |
| 5 | 74,5 | 16 | 99 | |
| 6 | 72,5 | 100 | | |

### Exemple 7 (comparatif)

Selon la technique du lit fluidisé, on enrobe 200 g de méthionine, préalablement granulée sous forme de particules sphériques titrant 98 % dont le diamètre est compris entre 0,5 et 0,6 mm par une solution, dans le tétrahydrofuranne, de 60 g d'une composition formée de 75 % d'éthylcellulose et de 25 % de copolymère vinyl-2 pyridine-styrène (70 - 30).

Des essais de relargage de la méthionine sont effectués en milieu aqueux à pH = 2 et à pH = 6 à 40° C sous agitation et à des intervalles de temps différents.

Les résultats sont donnée dans le tableau 4.

### Exemple 8

On opère comme dans l'exemple 7 mais en utilisant une composition d'enrobage constituée de 45 % d'éthylcellulose, de 30 % de dipropylèneglycol et de 25 % de copolymère vinyl-2 pyridine-styrène (70 - 30).

Les résultats sont donnés dans le tableau 4.

### Exemple 9

On opère comme dans l'exemple 7 mais en utilisant une composition d'enrobage constituée de 37,5 % d'éthylcellulose, de 37,5 % de dipropylène glycol et de 25 % de copolymère vinyl-2 pyridine-styrène (70 - 30).

Les résultats sont donnés dans le tableau 4.

### Exemple 10

On opère comme dans l'exemple 7 mais en utilisant une composition d'enrobage constituée de 40 % d'éthylcellulose, 40 % de propylène glycol et de 20 % de copolymère vinyl-2 pyridine-styrène (70-30).

Les résultats sont donnés dans le tableau 4.

### Exemple 11

On opère comme dans l'exemple 7 mais en utilisant une composition d'enrobage constituée de 45 % d'éthylcellulose, de 30 % de propylène glycol et de 25 % de copolymère vinyl-2 pyridine-styrène (70 - 30).

Les résultats sont donnés dans le tableau 4.

### Exemple 12 (comparatif)

On opère comme dans l'exemple 7 mais en utilisant une composition d'enrobage constituée de 75 % d'éthylcellulose et de 25 % de copolymère méthyl-2 vinyl-5 pyridine-styrène (80 - 20).

Les résultats sont donnés dans le tableau 4.

**Exemple 13**

On opère comme dans l'exemple 7 mais en utilisant une composition d'enrobage constituée de 41,25 % d'éthylcellulose, 33,75 % de propylèneglycol et de 25 % de copolymère méthyl-2 vinyl-5 pyridine-styrène (80 - 20).

Les résultats sont donnés dans le tableau 4.

**Exemple 14** (comparatif)

On opère comme dans l'exemple 7 mais en utilisant une composition d'enrobage constituée de 80 % d'éthylcellulose et de 20 % de copolymère méthyl-2 vinyl-5 pyridine-styrène (80 - 20).

Les résultats sont donnés dans le tableau 4.

**Exemple 15**

On opère comme dans l'exemple 7 mais en utilisant une composition d'enrobage constituée de 40 % d'éthylcellulose, 40 % de propylèneglycol et 20 % de copolymère méthyl-2 vinyl-5 pyridine-styrène (80 - 20).

Les résultats sont donnés dans le tableau 4.

**Tableau 4**

| Exemples | % de méthionine relarguée à pH = 2 après: | | | | % de méthionine relarguée à pH = 6 après: | | | | |
|---|---|---|---|---|---|---|---|---|---|
|  | 30 mm | 1 h. | 2 h. | 5 h. | 1 h. | 2 h. | 3 h. | 5 h. | 24 h. |
| 7 |  | 3 | 5 | 9 | 2 | 3 | 4 | 5 |  |
| 8 | 44 | 78 | 100 |  |  | 1 |  | 2 |  |
| 9 | 96 | 100 |  |  | 2 | 2 | 3 | 6 |  |
| 10 | 5 | 23 | 76 | 100 | 1 | 2 | 3 | 5 | 11 |
| 11 | 10 | 50 | 92 | 100 | 1 | 2 | 3 | 4 |  |
| 12 |  | 9 |  | 50 |  |  |  | 3 | 15 |
| 13 | 98 |  |  |  |  |  |  | 5 | 10 |
| 14 | 2 |  |  | 20 |  |  |  | 3 | 5 |
| 15 |  | 96 |  |  |  |  |  | 9 | 17 |

**Exemple 16** (comparatif)

Selon la technique du lit fluidisé on enrobe 200 g de monochlorhydrate de lysine, préalablement granulé sous forme de particules sphériques titrant 85 % dont le diamètre est compris entre 1 et 1,25 mm, par une solution, dans le tétrahydrofuranne, de 200 g d'une composition constituée de 75 % d'éthylcellulose et de 25 % de copolymère vinyl-2 pyridine-styrène (70 - 30).

Les essais de relarguage du monochlorhydrate de lysine sont effectués en milieu aqueux à pH = 2 et à pH = 6 à 40°C sous agitation et à des intervalles de temps différents.

Les résultats sont donnés dans le tableau 5.

**Exemple 17**

On opère comme dans l'exemple 16 mais en utilisant une composition d'enrobage constituée de 37,5 % d'éthylcellulose, de 37,5 % de propylène glycol et de 25% de copolymère vinyl-2 pyridine-styrène (70 - 30).

Les résultats sont donnés dans le tableau 5.

**Tableau 5**

| Exemples | % de monochlorhydrate de lysine relargué à pH = 2 | | | | | % de monochlorhydrate de lysine relargué à pH = 6 après: | | | | |
|---|---|---|---|---|---|---|---|---|---|---|
| | 30 mm | 1 h. | 2 h. | 3 h. | 5 h. | 1 h. | 2 h. | 3 h. | 5 h. | 24 h. |
| 16 | 2 | 2 | 2 | 2 | 7 | 2 | 2 | 2 | 5 | |
| 17 | 96 | 100 | | | | | | 1 | 6 | 65 |

**Exemple 18** (comparatif)

Selon la technique du lit fluidisé, avec une cuve equipée d'un système WURSTER, on enrobe 350 g de méthionine, préalablement granulée sous forme de particules sphériques titrant 98 % et dont le diamètre moyen est compris entre 0,5 et 0,63 mm, par une solution/dispersion dont la composition en poids est la suivante:

| | |
|---|---|
| Ethylcellulose (qualité N 22 de Hercules) | 90 g |
| Triacétine | 10 g |
| Dichloro-1,2 éthane | 500 cm³ |
| Ethanol | 500 cm³ |
| Antistatique (Labrasol marque déposée Gattefossé) | 3 cm³ |

Au bout de 3 heures, on obtient des granulés régulièrement enrobés titrant 73,6 % en méthionine.

Le relargage de la méthionine est déterminé en dispersant 8 g de granulés ainsi préparés dans 1 litre d'une solution tamponnée à 40°C agitée magnétiquement et maintenue à pH constant.

La quantité de méthionine relarguée est contrôlée sur des prélèvements; les essais étant effectués à pH = 6 et à pH = 2.

Les résultats sont rassemblés dans le tableau 6.

**Exemple 19**

On opère comme dans l'exemple 18 mais en utilisant une solution/dispersion enrobante dont la composition est la suivante:

| | |
|---|---|
| Zèine | 90 g |
| Triacétine | 10 g |
| Dichloco-1,2 éthane | 500 cm³ |
| Ethanol | 500 cm³ |
| Antistatique | 3 cm³ |

Les résultats sont rassemblés dans le tableau 6.

**Exemple 20**

On opère comme dans l'exemple 18, mais en ajoutant à la composition d'enrobage 20 g d'un copolymère styrène-vinyl-2 pyridine (29,5 - 69,5 en poids) de viscosité spécifique 0,592 mesurée à la concentration de 5 g/litre dans le diméthylformamide à 20°C.

Les résultats sont rassemblés dans le tableau 6.

**Exemple 21**

On opère commdans l'exemple 18 mais en ajoutant à la composition d'enrobage 20 g d'un copolymère styrène-vinyl-2 pyridine identique à celui utilisé dans l'exemple 20.

Les résultats sont rassemblés dans le tableau 6.

**Exemple 22** (comparatif)

On opère comme dans l'exemple 18 mais en utilisant une composition d'enrobage ayant la composition suivante:

| | |
|---|---|
| Zéine | 80 g |
| Ethylcellulose | 20 g |
| Acétate de polyvinyle | 10 g |
| Triacétine | 10 g |
| Dichloro-1,2 éthane | 500 cm$^3$ |
| Ethanol | 500 cm$^3$ |
| Antistatique (Labrasol) | |
| | 3 cm$^3$ |

Les résultats sont rassemblés dans le tableau 6.


**Exemple 23**

On opère comme dans l'exemple 22 mais en ajoutant à la solution d'enrobage 20 g de copolymère styrène-vinyl-2 pyridine identique à celui utilisé dans l'exemple 20.
Les résultats sent rassemblés dans le tableau 6.

**Tableau 6**

| Exemple | Titre en méthionone | Relargage à pH = 6 | | Relargage à pH = 2 | |
|---|---|---|---|---|---|
| | | après 6 h. | après 24 h. | après 2 h. | après 5 h. |
| 18 | 79,7 | 2,5 | 8 | 2,8 | 4,2 |
| 19 | 78 | 90 | 100 | 100 | |
| 20 | 82,7 | 32,5 | 75,5 | 50 | 91 |
| | | | | 67 en 8 mn | |
| 21 | 72 | 10 | 27 | | |
| | | | | 92 en 30 mn | |
| 22 | 74,3 | 13 | 17 | 8,4 | 22 |
| 23 | 73,5 | 23 | 25 | 97 en 15 mn | |


**Exemple 24**

Selon la technique du lit fluidisé, avec une cuve équipée d'un système WURSTER, on enrobe 350 g de méthionine, préalablement granulée sous forme de particules sphériques titrant 98 % et dont le diamètre moyen est compris entre 0,5 et 0,63 mm, par une solution/dispersion dont la composition en poids est la suivante:

| | |
|---|---|
| Zéine | 60 g |
| Ethylcellulose (qualité N22 de Hercules) | 30 g |
| Triacétine | 10 g |
| Copolymère vinyl-2 pyridine-styrène (70 - 30) | 20 g |
| Dichloro-1,2 éthane | 500 cm$^3$ |
| Ethanol | 500 cm$^3$ |
| Antistatique (Labrasol, marque déposée Gattefossé) | 3 cm$^3$ |

La viscosité spécifique du copolymère vinyl-2 pyridine-styrène est de 0,560 mesurée à la concentration de 5 g/litre dans le diméthylformamide à 20°C.
Au bout de 3 heures, on obtient des granulés régulièrement enrobés titrant 72,1 % en méthionine.
Le relargage de la méthionine est déterminé dans les conditions décrites dans l'exemple 18.
Les résultats sont rassemblés dans le tableau 7.

**Exemples 25**

On opère comme dans l'exemple 24 mais en ajoutant seulement 10 g de copolymère vinyl-2 pyridine-styrène (70 - 30).

Les résultats sont rassemblés dans le tableau 7.

**Exemple 26**

On opère comme dans l'exemple 25 mais en utilisant un copolymère vinyl-2 pyridine-styrène (70 - 30) dont la viscosité spécifique est 0,610.

Les résultats sont rassemblés dans le tableau 7.

**Exemple 27**

On opère comme dans l'exemple 25 mais en remplaçant le copolymère vinyl-2 pyridine-styrène (70 - 30) par un copolymère méthyl-2 vinyl-5 pyridine-styrène (77,5 - 22,5) dont la viscosité spécifique est 0,496.

Les résultats sont rassemblés dans le tableau 7.

**Exemple 28**

On opère comme dans l'exemple 25 mais en remplaçant le copolymère vinyl-2 pyridine-styrène (70 - 30) par un homopolymère vinyl-2 pyridine.

Ces résultats sont rassemblés dans le tabelau 7.

**Exemple 29**

On opère comme dans l'exemple 25 mais en remplaçant le copolymère vinyl-2 pyridine-styrène (70 - 30) par un copolymère vinyl-2 pyridine-styrène (90 - 10).

Les résultat sont rassemblés dans le tableau 7.

**Exemple 30**

On opère comme dans l'exemple 25 mais en remplaçant le copolymère vinyl-2 pyridine-styrène (70 - 30) par un copolymère vinyl-2 pyridine-méthacrylate d'hydroxy-2 éthyle réticulé.

Les résultats sont rassemblés dans le tableau 7.

**Tableau 7**

| Exemples | Titre en méthionine % | Relargage à pH = 6 | | Relargage à pH = 2 après | | | |
|---|---|---|---|---|---|---|---|
| | | après 6 h. % | après 24 h. % | 15 mm % | 30 mm % | 1 h. % | 2 h. % |
| 24 | 72,1 | 10 | 26 | 100 | | | |
| 25 | 72,0 | 10 | 18 | 63 | 100 | | |
| 26 | 74,9 | 2,0 | 6,8 | 35 | 85 | 100 | |
| 27 | 74,4 | 4,5 | 8,6 | 69 | 94 | 100 | |
| 28 | 74,0 | 2,3 | 4,5 | 3,8 | 5,0 | 8,1 | 12,6 |
| 29 | 75,2 | 3,3 | 6,7 | 14,9 | 26,5 | 40,3 | 77,4 |
| 30 | 74,6 | 2,2 | 3,7 | | | 3,6 | 13,7 |

**Exemple 31**

Selon la technique du lit fluidisé, avec une cuve équipée d'un système WURSTER, on enrobe 350 g de chlorhydrate de lysine, préalablement granulée sous forme de particules sphériques titrant 85 % et dont le diamètre moyen est compris entre 0,63 et 0,8 mm par une solution/dispersion dont la composition est identique à celle décrite dans l'exemple 25.

Le relargage du chlorhydrate de lysine est déterminé dans les conditions décrites dans l'exemple 18.

Les résultats sont rassemblés dans le tableau 8.

**Exemple 32**

On opère comme dans l'exemple 31 mais en augmentant le taux d'enrobant, la composition d'enrobage étant la même.

Les résultats sont rassemblés dans le tableau 8.

**Exemple 33**

On opère comme dans l'exemple 31 mais en utilisant une composition d'enrobage constituée de:

| | |
|---|---|
| Zéine | 82 g |
| Ethylcellulose (qualité N22 Hercules) | 41 g |
| Triacétine | 13,7 g |
| Copolymère vinyl-2 pyridine-styrène (70 - 30) | 13,7 g |
| Aluminium (pâte STAPA 4 n1 à 65 % d'aluminium) | 100 g |
| Dichloro-1,2 éthane | 683 cm$^3$ |
| Ethanol | 683 cm$^3$ |
| Antistatique (Labrasol marque déposée Gattefossé) | 3 cm$^3$ |

Les résultats sont rassemblés dans le tableau 8.

**Exemple 34**

On opère comme dans l'exemple 32 mais en remplaçant 100 g de pâte STAPA 4 n1 par 230 g de la même pâte de façon à avoir 150 g d'aluminium dans la composition d'enrobage.

Les résultats sont rassemblés dans le tableau 8

**Exemple 35**

On opère comme dans l'exemple 34 mais en remplaçant le copolymère vinyl-2 pyridine-styrène (70 - 30) par un copolymère méthyl-2 vinyl-5 pyridine-styrène (77,5 - 22,5) dont la viscosité spécifique est 0,496.

Les résultats sont rassemblés dans le tableau 8.

**Tableau 8**

| Exemples | Titre en lysine, Hc1 % | Relargage à pH = 6 | | Relargage à pH = 2 après: | | |
|---|---|---|---|---|---|---|
| | | après 6 h. % | après 24 h. % | 15 mm % | 30 mm % | 1 h. % |
| 31 | 67,5 | 100 | | 100 | | |
| 32 | 51,2 | 13,2 | 75,5 | 37,4 | 100 | |
| 33 | 50,2 | 40,7 | 88,6 | 57,9 | 100 | |
| 34 | 47,5 | 4,7 | 60,2 | 34,6 | 80,3 | 100 |
| 35 | 46,8 | 5,8 | 61,1 | 32,4 | 81,2 | 100 |

**Revendications**

1. Une composition pour l'enrobage d'une substance biologiquement active qui est stable à un pH supérieur ou égal à 5 et qui libère la substance active à un pH inférieur ou égal à 3,5 caractérisée en ce qu'elle est constituée:
- de 40 à 95 % en poids d'un système liant à hydrophilie contrôlée qui est éventuellement légèrement sensible aux variations du pH qui est choisi parmi l'association d'un dérivé de la cellulose filmogène non hydrosoluble et d'un agent de contrôle de la balance hydrophile/hydrophobe choisi parmi les polyols dans laquelle le rapport du dérivé de la cellulose au polyol est compris entre 1/1 et 1,5/1 ; la zéine ; et une association de zéine et d'un agent de contrôle de la balance hydrophile/hydrophobe choisi parmi les dérivés de la cellulose non hydrosolubles dans le rapport 2/1 à 10/1,
- et de 60 à 5 % en poids d'une substance sensible aux variations du pH choisi parmi les polymères ou copolymères contenant au moins un groupe aminobasique et dont la teneur en azote est comprise entre 2 et 14 % choisis parmi les dérivés aminés de la cellulose, les polymères des dérivés aminés des acides acrylique, méthacrylique et crotonique et les polymères du styrène ou de l'acrylonitrile avec les isomères ou les dérivés de la vinylpyridine, et le chitosan.
2. Une composition selon la revendication 1 caractérisée en ce qu'elle contient en outre un agent plastifiant
3. Une composition selon la revendication 2 caractérisée en ce que, le liant contenant de la zéine, on utilise comme plastifiant la triacétine.
4. Une composition selon la revendication 1 caractérisée en ce qu'elle contient en outre un polymère filmogène hydrophobe additionnel.
5. Une composition selon la revendication 4 caractérisée en ce que le polymère filmogène hydrophobe est l'acétate de polyvinyle.
6. Une composition selon la revendication 1 caractérisée en ce que le polyol est choisi parmi le glycérol, l'éthylène glycol, le propylène glycol ou le dipropylène glycol.
7. Une composition selon la revendication 1 caractérisée en de qu'elle contient en outre des charges lamellaires choisies parmi le mica, le talc et l'aluminium.
8. Une composition selon la revendication 1 caractérisée en ce que le liant est constitué par une association de zéine et d'éthylcellulose et la substance sensible aux variations du pH est le chitosan.
9. Une composition selon la revendication 1 caractérisée en ce que le liant est constitué par une association de zéine et d'éthylcellulose et la substance sensible aux variations du pH est un polymère ou copolymère basique.
10. Granulés destinés à être administrés par voie orale à des animaux, caractérisés en ce qu'ils sont constitués d'un noyau contenant une substance biologiquement active protégée par une composition d'enrobage selon l'une des revendications 1 à 9.
11. Granulés selon la revendication 10 caractérisés en ce que la composition d'enrobage représente 5 à 60 % en poids du granulé enrobé.
12. Granulés selon la revendication 10 ou 11 caractérisés en ce que la substance biologiquement active est choisie parmi les médicaments, les vitamines et les amino acides.
13. Granulés selon la revendication 12 caractérisés en ce que la substance active est choisie parmi la lysine ou la méthionine.

**Patentansprüche**

1. Eine Masse zur Umhüllung einer biologisch aktiven Substanz, welche Masse bei einem pH-Wert größer oder gleich 5 stabil ist und die aktive Substanz bei einem pH-Wert kleiner oder gleich 3,5 freigibt, dadurch gekennzeichnet, daß sie besteht aus:
- 40 bis 95 Gew.-% eines kontrolliert hydrophilen Bindemittelsystems, welches gegebenenfalls leicht empfindlich gegenüber PH-Veränderungen ist und ausgewählt ist aus einer Kombination eines nicht wasserlöslichen filmbildenden Cellulosederivats mit einem Mittel zur Kontrolle des Gleichgewichts hydrophil/hydrophob, ausgewählt aus den Polyolen, wobei das Verhältnis von Cellulose zu Polyol zwischen 1/1 und 1,5/1 beträgt; aus Zein; und aus einer Kombination von Zein und einem Mittel zur Kontrolle des Gleichgewichts hydrophil/hydrophob, ausgewählt aus den nicht wasserlöslichen Cellulosederivaten, im Verhältnis 2/1 bis 10/1,

- und 60 bis 5 Gew.-% einer gegenüber pH-Veränderungen empfindlichen Substanz, ausgewählt aus den Polymeren oder Copolymeren, die zumindest eine aminobasische Gruppe enthalten und deren Stickstoffgehalt 2 bis 14 % beträgt, ausgewählt aus den Aminoderivaten von Cellulose, den Polymeren von Aminoderivaten von Acryl-, Methacryl- und Krotonsäure und den Polymeren von Styrol oder Acrylnitril mit den Isomeren oder den Derivaten von Vinylpyridin und Chitosan.

2. Masse nach Anspruch 1, dadurch gekennzeichnet, daß sie außerdem einen Weichmacher enthält.

3. Masse nach Anspruch 2, dadurch gekennzeichnet, daß man mit einem Zein enthaltenden Bindemittel als Weichmacher Triacetin verwendet.

4. Masse nach Anspruch 1, dadurch gekennzeichnet, daß sie außerdem ein zusätzliches hydrophobes filmbildendes Polymer enthält.

5. Masse nach Anspruch 4, dadurch gekennzeichnet, daß das hydrophobe filmbildende Polymer Polyvinylacetat ist.

6. Masse nach Anspruch 1, dadurch gekennzeichnet, das das Polyol ausgewählt ist aus Glycerin, Äthylenglykol, Propylenglykol oder Dipropylenglykol.

7. Masse nach Anspruch 1, dadurch gekennzeichnet, daß sie außerdem eine lamellenförmige Charge, ausgewählt aus Glimmer, Talkum und Aluminium, enthält.

8. Masse nach Anspruch 1, dadurch gekennzeichnet, daß das Bindemittel aus einer Mischung von Zein und Äthylcellulose besteht und daß die gegenüber pH-Veränderungen empfindliche Substanz Chitosan ist.

9. Masse nach Anspruch 1, dadurch gekennzeichnet, daß das Bindemittel aus einer Mischung von Zein und Äthylcellulose besteht und daß die gegenüber pH-Veränderungen empfindliche Substanz ein basisches Polymer oder Copolymer ist.

10. Granulate für die orale Verabreichung an Tiere, dadurch gekennzeichnet, daß sie aus einem eine biologisch aktive Substanz enthaltenden Kern bestehen, der durch eine Umhüllungsmasse nach einem der Ansprüche 1 bis 9 geschützt ist.

11. Granulate nach Anspruch 10, dadurch gekennzeichnet, daß die Umhüllungsmasse 5 bis 60 Gew.-% des umhüllten Granulats ausmacht.

12. Granulats nach Anspruch 10 oder 11, dadurch gekennzeichnet, daß die biologisch aktive Substanz ausgewählt ist aus den Medikamenten, den Vitaminen und den Aminosäuren.

13. Granulate nach Anspruch 12, dadurch gekennzeichnet, daß die aktive Substanz ausgewählt ist aus Lysin oder Methionin.

## Claims

1. A composition for coating a biologically active substance which is stable at a pH higher than or equal to 5 and which releases the active substance at a pH lover than or equal to 3.5, characterized in that it consists:

- of 40 to 95 % by weight of a binder system with controlled hydrophilicity which, if desired, is slightly sensitive to pH variations which is chosen from the combination of a derivative of water-insoluble film-forming cellulose and an agent for controlling the hydrophilic/hydrophobic balance, chosen from poluols in which the ratio of the cellulose derivative to the polyol is between 1/1 and 1.5/1, zein, and a combination of zein and of an agent for controlling the hydrophilic/hydrophobic balance chosen from water-insoluble cellulose derivatives in the ratio 2/1 to 10/1,

- and of 60 to 5 % by weight of a substance which is sensitive to pH variations, chosen from polymers or copolymers containing at least one aminobasic group and whose nitrogen content is between 2 and 14 % which are chosen from the amino derivatives of cellulose, polymers of amino derivatives of acrylic, methacrylic and crotonic acids and polymers of styrene or of acrylonitrile with the isomers or derivatives of vinylpyridine, and chitosan.

2. A composition according to Claim 1, characterized in that it additionally contains a plasticizing agent.

3. A composition according to claim 2 characterized in that triacetin is employed as a plasticizer, the binder containing zein.

4. A composition according to Claim 1, characterized in that it also contains an additional hydrophobic film-forming polymer.

5. A composition according to Claim 4, characterized in that the hydrophobic film-forming polymer is polyvinyl acetate.

6. A composition according to Claim 1, characterized in that the polyol is chosen from glycerol, ethylene glycol, propylene glycol or dipropylene glycol.

7. A composition according to Claim 1, characterized in that it additionally contains lamellar fillers chosen from mica, talc and aluminium.

8. A composition according to Claim 1, characterized in that the binder consists of a combination of zein and of ethyl cellulose and the substance which is sensitive to pH variations is chitosan.

9. A composition according to Claim 1, characterized in that the binder consists of a combination of zein and ethyl cellulose and the substance which is sensitive to pH variations is a basic polymer or copolymer.

10. Granules intended to be administered orally to animals, characterized in that they cons ist of a core containing a biologically active substance protected by a coating composition according to one of Claims 1 to 9.

11. Granules according to Claim 10, characterized in that the coating composition represents 5 to 60 % by weight of the coated granule.

12. Granules according to Claim 10 or 11, characterized in that the biologically active substance is chosen from medicines, vitamins and amino acids.

13. Granules according to Claim 12, characterized in that the active substance is chosen from lysine or methionine.